# EUROPEAN PATENT APPLICATION

(11) **EP 1 262 194 A1**
(43) Date of publication of application: **04.12.2002**
(21) Application number: 01908214.8
(22) Date of filing: 01.03.2001
(51) Int. Cl.: A61K 45/00, A61K 39/395, A61K 38/17, A61P 43/00, A61P 7/02, A61P 15/08

(54) **REMEDIES AND PREVENTIVES FOR ANTIPHOSPHOLIPID ANTIBODY SYNDROME**

(30) Priority: 06.03.2000 JP 2000059949
(71) Applicant: Eisai Co., Ltd., Tokyo 112-8088 (JP)
(72) Inventor: YAMAUCHI, Toshihiko, Tsukuba-shi, Ibaraki 305-0045 (JP); YOKOHAMA, Hiromitsu, Moriya-shi, Ibaraki 302-0127 (JP); KOBAYASHI, Seiichi, Belmont, MA 02478 (US); SETO, Toshio, Ushiku-shi, Ibaraki 300-1233 (JP)
(74) Representative: Bannerman, David Gardner
(86) International application number: JP0101585
(87) International publication number: WO01066141

(57) **Abstract**

Agents for treating and preventing antiphospholipid antibody syndrome (APS) comprising, as an active ingredient, a substance inhibiting interaction between gp39 on a T cell surface, which is a receptor mediating contact-dependent helper effector function, and CD40 on an antigen-presenting cell surface.

## Description

### TECHNICAL FIELD

The present invention relates to agents for treating antiphospholipid antibody syndrome (APS) for administrating to APS patients, and agents for preventing afflication of APS for prophylactic administration to patients of autoimmune diseases such as systemic lupus erythematosus (SLE) who are expected to be afflicted by APS.

### BACKGROUND ART

APS is a generic name of diseases in which antibodies for phospholipid such as cardiolipin are positive. APS is often observed in SLE but underlying diseases are recognized to be various. In some cases, no underlying disease is recognized and the case is named as primary antiphospholipid antibody syndrome. It is known that APS causes arterial thrombosis in a brain, a heart and extremities, thrombocytopenia and habitual abortion.

Concerning the treatment of APS, antithrombotic treatment using antithrombotic drugs such as heparin and warfarin is the major treatment method.

Interaction between a gp39 molecule on a CD4⁺ Th cell surface and a CD40 molecule on an antigen-presenting cell surface is essential for activation of the CD4⁺ Th cell by recognition of an antigen presented on an MHC class II molecule on the antigen-presenting cell surface by the CD4⁺ Th cell. When this interaction is inhibited by a gp39 antagonist, for example, an anti-gp39 antibody, unresponsive T cell tolerance is induced in the CD4⁺ Th cell (WO95/06481).

Similarly, interaction between a gp39 molecule on a CD4⁺ Th cell surface and a CD40 molecule on a B cell surface is essential for differentiation of B cell into an antibody-producing cell upon stimulation by an antigen. It is known that antibody production is blocked when this interaction is inhibited by a gp39 antagonist, for example, an anti-gp39 antibody (WO95/06480).

Since a gp39 antagonist inhibits both of immune responses of the CD4+ Th cell and the B cell, attempts are being made to apply gp39 antagonists to treatment of autoimmune diseases (SLE, idiopathic thrombocytopenic purpura (ITP) and so forth), in which pathological conditions are developed by immune responses to autoantigens. Concerning APS, however, the effect of administration of the gp39 antagonist has not been discussed in any report.

### DISCLOSURE OF THE INVENTION

An object of the present invention is to develop an agent for the treatment of APS which is administered to APS patients, and an agent for preventing affliction of APS which is administrated to patients of autoimmune diseases such as systemic lupus erythematosus (S_{L}E) who are expected to be afflicted by APS.

As a result of studies based on an idea that the treatment to reduce the production of the antiphospholipid antibody is not a direct treatment in the point of suppressing the thrombosis, but is worth considering as a fundamental treating and preventing method to reduce the amount of the antiphospholipid antibody by which the thrombus is caused, the inventors of the present invention revealed that the production of the anti-cardiolipin antiboby is suppressed and necrosis of cardiac muscle accompanying APS is effectively prevented by administering an anti-gp39 antibody, which is a gp39 antagonist, to a (NZW x BXSB) F₁ mouse, which genetically develops APS-like pathological condition (thrombocytopenia and anti-cardiolipin antibody production) with aging. Thus, they accomplished the present invention.

The present invention provides an agent for treating APS comprising, as an active ingredient, a substance inhibiting interaction between gp39 on a T cell surface, which is a receptor mediating contact-dependent helper effector function, and CD40 on an antigen-presenting cell surface, and an agent for preventing APS comprising, as an active ingredient, a substance inhibiting interaction between gp39 on a T cell surface, which is a receptor mediating contact-dependent helper effector function, and CD40 on an antigen-presenting cell surface. The substance inhibiting the interaction is preferably an anti-gp39 antibody.

The present invention also provides a method for treating APS comprising administering a substance inhibiting interaction between gp39 on a T cell surface, which is a receptor mediating contact-dependent helper effector function, and CD40 on an antigen-presenting cell surface, to an APS patient, and a method for preventing APS comprising administrating a substance inhibiting interaction between gp39 on the T cell surface, which is a receptor mediating contact-dependent helper effector function, and CD40 on an antigen-presenting cell surface to a patient who is expected to be afflicted by APS. The substance inhibiting the interaction is preferably an anti-gp39 antibody.

The present invention further provides use of a substance inhibiting interaction between gp39 on a T cell surface, which is a receptor mediating contact-dependent helper effector function, and CD40 on an antigen-presenting cell surface for manufacture of an agent for treating APS, and use of a substance inhibiting interaction between gp39 on a T cell surface, which is a receptor mediating contact-dependent helper effector function, and CD40 on an antigen-presenting cell surface for manufacture of an agent for preventing APS. The substance inhibiting the interaction is preferably an anti-gp39 antibody.

The method for inhibiting the antiphospholipid antibody production by administration of a substance inhibiting interaction between gp39 on a T cell surface, which is a receptor mediating contact-dependent helper effector function, and CD40 on an antigen-presenting cell surface, such as a gp39 antagonist, to an APS patient, or by prophylactic administration of a substance inhibiting interaction between gp39 on a T cell surface, which is a receptor mediating contact-dependent helper effector function, and CD40 on an antigen-presenting cell surface, to an patient of autoimmune diseases such as SLE, who is expected to be afflicted by APS, is predicted to be a fundamental treating method of APS.

### BRIEF DESCRIPTION OF THE DRAWINGS

Fig. 1 shows a suppressive effect of MR1 on anti-cardiolipin antiboby production of (NZW x BXSB) F₁ mice. ▲: MR1-administered group (500 µg/mouse), Δ: Control group. The values are shown as mean value for surviving mice.
Fig. 2 shows a suppressive effect of MR1 on necrosis of cardiac muscle in (NZW x BXSB) F₁ mice.

### BEST MODE FOR CARRYING OUT THE INVENTION

Various embodiments of the present invention will be explained in detail below with respect to the following items.

### 1. gp39 antagonist

A gp39 antagonist is defined as a substance inhibiting interaction between gp39 on a T cell surface, which is a receptor mediating contact-dependent helper effector function, and CD40 on a antigen-presenting cell surface. The gp39 antagonists include not only substances interacting with gp39, but also substances interacting with CD40. The gp39 antagonist may be an antibody directed to gp39 (for example, monoclonal antibodies directed to gp39), a fragment of the antibody directed to gp39 (for example, Fab or F(ab')₂ fragment), a chimera antibody or a humanized antibody, soluble CD40 or soluble CD40L and a fragment thereof, or any other compounds inhibiting the interaction between gp39 and CD40.

The property of the gp39 antagonist for inhibiting the interaction between gp39 and CD40 can be determined based on, for example, if it inhibits binding of a labeled soluble CD40 to an activated helper T cell. The labeled soluble CD40 can be produced by preparing soluble CD40 using, for example, the method described in Example 1 in Japanese Patent Application Laid-open No. 6-220096 and labeling the soluble CD40 with an appropriate labeling substance, for example, a fluorescent substance, a radioisotope or the like. The activated helper T cell can be prepared by, for example, activation with an anti-CD3 antibody or the like according to the method described in Example 1 in Japanese Patent Application Laid-open No. 6-220096. Binding of the labeled soluble CD40 to the activated helper T cell can be evaluated by FACS using, for example, a fluorescence-labeled soluble CD40.

### 2. Anti-gp39 antibody

A mammal (for example, mouse, hamster or rabbit) can be immunized with a gp39 protein or a protein fragment thereof (for example, peptide fragments) in the form of an immunogen that induces immune responses in the mammal.

The gp39 protein can be obtained by allowing expression of an expression vector to which gp39 cDNA (Armitage et al., Nature, 357: 80-82, 1992; Hollembaugh et al., EMBO J., 11: 4313-4319, 1992) is inserted, in a host cell, for example, a bacterial or mammalian cell strain, and purifying the protein from a culture broth of the cell according to a standard method. The gp39 protein may be expressed as, for example, a fusion protein with GST or the like. A fusion protein with GST may be purified by using a glutathione column. The gp39 peptide can be synthesized by a known method (for example, F-moc or T-boc chemical synthesis) based on the amino acid sequence of gp39 (Armitage et al., Nature, 357: 80-82, 1992; Hollembaugh et al., EMBO J., 11: 4313-4319, 1992). Immunogenicity of the synthesized peptide can be improved by binding the peptide to an appropriate carrier, for example, KLH.

Antiserum can be obtained after immunization with the purified gp39 protein or the peptide fragment thereof together with an adjuvant. If desired, polyclonal antibodies can be isolated from the antiserum. To produce monoclonal antibodies, antibody-producing cells (lymphocytes) are recovered from an immunized animal and immortalized by fusing them with myeloma cells according to a standard cell fusion method to obtain hybridomas. This technique is an established method in this field and can be performed according to descriptions of an appropriate manual (Harlow et al., Antibodies: A Laboratory Manual, 1998, Cold Spring Harbor Laboratory). Further, monoclonal antibodies may also be prepared by other methods including the human B cell hybridoma method for producing human monoclonal antibodies (Kozbar et al., Immunol. Today, 4: 72, 1983), the EBV-hybridoma method (Cole et al., Monoclonal Antibody in Cancer Therapy, 1985, Allen R. Bliss, Inc., pages 77-96), screening of combinatorial antibody library (Huse et al., Science, 246: 1275, 1989) and so forth.

The antibodies referred to in the present specification include fragments of antibodies specifically binding to gp39, for example, Fab and (Fab')₂ fragments.

Monoclonal antibodies prepared by using an animal other than human, e.g., mouse monoclonal antibodies prepared by using mouse as an immunized animal, are often recognized as heterogenous proteins when administered to human, and therefore an immune response to the antibodies may be often induced. A solution to this problem is provided by chimera antibodies, that is, antibodies having an antigen-binding region derived from a mouse monoclonal antibody and other regions derived from a human antibody. The antibodies used in the present invention also include such chimera antibodies. Examples of the chimera antibodies include chimera antibodies using the whole variable regions of a mouse monoclonal antibody as an antigen-binding region (Morrison et al., Proc. Natl. Acad. Sci. USA, 81: 6851, 1985; Takeda et al., Nature, 314: 452, 1985), chimera antibodies utilizing a combination of a human-derived framework region and a hypervariable region derived from a mouse monoclonal antibody as an antigen-binding region (Teng et al., Proc. Natl. Acad. Sci. USA, 80: 7308-12, 1983; Kozbar et al., Immunol. Today, 4: 7279, 1983) and so forth. However, the present invention is not limited to these.

The agent for treating APS of the present invention can be administered to an APS patient. Also, the agent for preventing APS of the present invention can be administered to a patient expected to be afflicted by APS to prevent affliction of APS.

The agents for treating and preventing APS of the present invention can be administered by a usual method such as injection (subcutaneous, intravenous or the like).

The form of the agents for treating and preventing APS are appropriately selected depending on the administration method. Examples of pharmaceutical compositions suitable for use as injection include, for example, sterilized aqueous solutions (when the substance is water-soluble), dispersions, sterilized powders for immediately preparing sterilized injection solutions or dispersions. The pharmaceutical compositions suitable for use as injection must be sterilized and has sufficient fluidity for easy syringe operation in any case. The compositions must be stable under production and storage conditions and be protected from actions of contaminated microbes such as bacteria and fungi. A carrier may be a solvent or a dispersion medium, for example, water, ethanol, polyol (for example, glycerol, propylene glycol, polyethylene glycol etc.), an appropriate mixture thereof or the like. Appropriate fluidity can be maintained by using a coating such as one composed of lecithin, or maintaining a desired particle diameter or using a surfactant in case of dispersion. The protection from actions of microbes can be achieved by various antibacterial agents or antifungal agents, for example, paraben, chlorobutanol, phenol, ascorbic acid, thimerosal and so forth. In many cases, it is preferable to add isotonic agents, for example, saccharides and polyalcohols including mannitol and sorbitol, sodium chloride and so forth to the composition. Sustained absorption of compositions for injection can be attained by adding substances for retarding the absorption, for example, aluminum monostearate, gelatin or like to the compositions.

The solutions for injection can be prepared by mixing an active compound (gp39 antagonist and so forth) in a desired amount and, if required, one of the aforementioned ingredients or a combination thereof in an appropriate solvent and then sterilizing the mixture by filtration. In general, dispersions are prepared by mixing an active compound with a base dispersion medium and a sterilized medium containing other required ingredients selected from the above. In case of using sterilized powder for preparing sterilized injection solution, preferable methods are vacuum dehydration or lyophilization, and powder of the active ingredient and desired additional ingredients sterilized by filtration beforehand can be obtained.

Doses of the agent for treating APS are amounts sufficient to treat APS and can vary depending on age, sex, susceptibility to the substance, administration method, clinical history and so forth of patients. Also, doses of the agent for preventing APS are amounts sufficient to prevent affliction of APS and can vary depending on age, sex, susceptibility to the substance, administration method, clinical history and so forth of patients.

### EXAMPLES

The present invention will be described in more detail with reference to the following examples, but the present invention is not limited to these.

The anti-gp39 antibody MR1 (Noelle et al., Proc. Natl. Acad. Sci. USA, 89: 6550-54, 1992) used in the following examples is a monoclonal antibody directed to a mouse gp39 prepared in a hamster and is available from PharMingen (Catalog No.: PM-09020D or PM-09021D). The MR1-producing cell is available from American Type Culture Collection (ATCC) (ATCC No.: HB-11048).

With respect to the (NZW x BXSB) F₁ mice used, it is reported that an autoantibody directed to cardiolipin is observed (Akinori et al., Eur. J. Immunol., 28: 2694-2703, 1998). These mice are available from Japan SLC.

### Example 1: Suppressive effect of MR1 on the anti-cardiolipin antibody production of male (NZW x BXSB) F₁ mice

A suppressive effect of MR1 on the anti-cardiolipin antibody production of male (NZW x BXSB) F₁ mice was examined. MR1 (Noelle et al., Proc. Natl. Acad. Sci. USA, 89: 6550-54, 1992) was purified from a culture supernatant of MR1-producing cells presented by Dr. Noelle by using protein A, and used. MR1 and hamster IgG as a negative control were each intraperitoneally administered to the mice once a week at a dose of 500 µg/200 µL/administration from the age of 5-week old through 16-week old. About 300 µL of blood was collected from the fundus oculi into an EDTA-treated tube by using a heparin-treated hematocrit capillary tube at the ages of 5, 9, 13 and 17-week old under etherization.

The plasma isolated from the collected blood was allowed to react with cardiolipin fixed on a 96-well plate, and then measurement was carried out by ELISA utilizing detection with peroxidase-labeled anti-mouse IgG. The antibody titer of samples (A.U./ml) was calculated based on an antibody titer of a serum of a syngeneic mouse of 35-week old used as a standard serum, which was taken as 1000 U/ml.

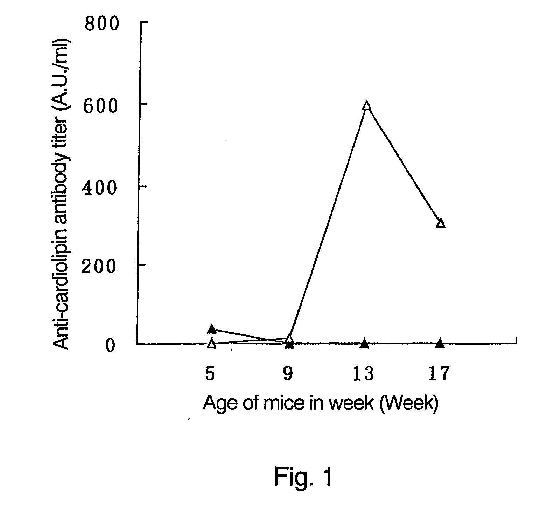

As a result, the anti-cardiolipin IgG antibody titer in plasma of the hamster IgG-administered control group increased from 9-week old and reached the peak at 13-week old. MR1 almost completely inhibited the increase of the autoantibody in blood until 17-weeks old at which the test was finished. A statistically significant difference was observed between the MR1-administered group and the control group (p < 0.05, Fig. 1).

### Example 2: Suppressive effect of MR1 on necrosis of cardiac muscle of (NZW x BXSB) F₁ mice

A suppressive effect of MR1 on necrosis of cardiac muscle in (NZW x BXSB) F₁ mice was examined. MR1 or hamster IgG as a negative control was intraperitoneally administered to mice once a week at a dose of 500 µg/200 µL/administration from the age of 5-week old through 16-week old. The cardiac muscle was taken at the age of 18-week old and pathological sample was prepared.

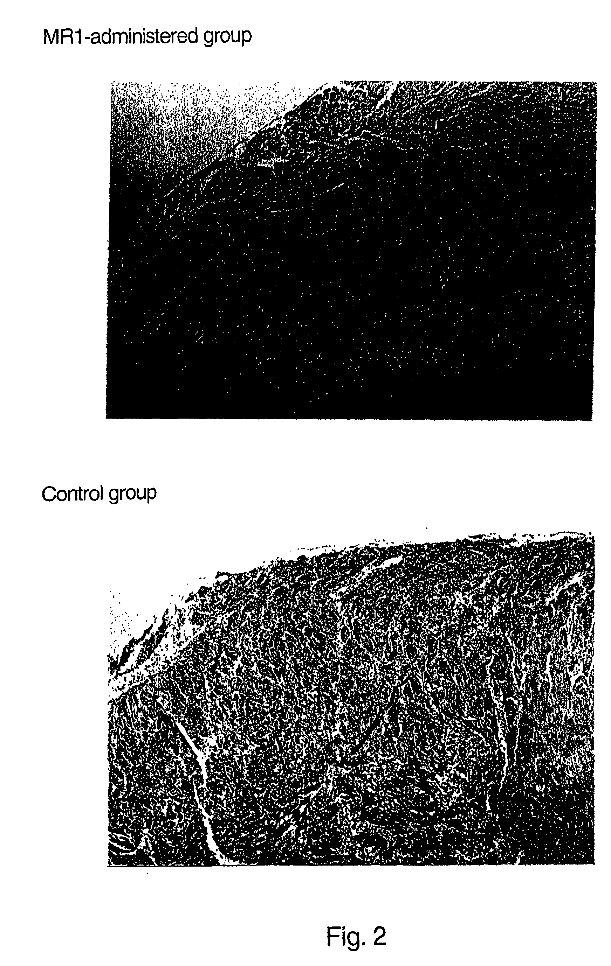

As a result, necrosis of cardiac muscle based on thrombosis was observed in two of four mice of the hamster IgG-administered control group. In the MR1-administered group (four mice), on the other hand, no necrosis of cardiac muscle was observed. Photographs of typical pathological samples are shown in Fig. 2.

### INDUSTRIAL APPLICABILITY

Based on the observation that the anti-cardiolipin antibody production is suppressed and necrosis of cardiac muscle by thrombosis is suppressed by administration of a gp39 antagonist, agents for treating and preventing APS containing, as an active ingredient, a gp39 antagonist are provided.

## Claims

1. An agent for treating antiphospholipid antibody syndrome (APS) comprising, as an active ingredient, a substance inhibiting interaction between gp39 on a T cell surface, which is a receptor mediating contact-dependent helper effector function, and CD40 on an antigen-presenting cell surface.

2. The agent for treating APS according to Claim 1, wherein the substance inhibiting the interaction is an anti-gp39 antibody.

3. An agent for preventing antiphospholipid antibody syndrome (APS) comprising, as an active ingredient, a substance inhibiting interaction between gp39 on a T cell surface, which is a receptor mediating contact-dependent helper effector function, and CD40 on an antigen-presenting cell surface.

4. The agent for preventing APS according to Claim 3, wherein the substance inhibiting the interaction is an anti-gp39 antibody.
